# EUROPÄISCHE PATENTSCHRIFT

(11) **EP 1 482 874 B1**
(45) Veröffentlichungstag und Bekanntmachung des Hinweises auf die Patenterteilung: **03.01.2007**
(21) Anmeldenummer: 02732467.2
(22) Anmeldetag: 08.03.2002
(51) Int. Cl.: A61F 2/42

(54) **SPRUNGGELENK-ENDOPROTHESE**
ANKLE-JOINT ENDOPROSTHESIS
ENDOPROTHESE D'ARTICULATION DE LA CHEVILLE

(43) Veröffentlichungstag der Anmeldung: 08.12.2004
(73) Patentinhaber: WALDEMAR LINK GmbH & Co. KG, 22339 Hamburg (DE)
(72) Erfinder: KOFOED, Hakon, DK--2920 Charlottenlund (DK); KELLER, Arnold, 23863 Kayhude (DE)
(74) Vertreter: Glawe, Delfs, Moll
(86) Internationale Anmeldenummer: PCT/EP2002/002573
(87) Internationale Veröffentlichungsnummer: WO 2003/075802

(56) Entgegenhaltungen:
- EP-A- 1 097 680
- WO-A-00/69373
- WO-A-01/89427
- DE-U- 8 812 806
- FR-A- 2 676 917
- FR-A- 2 684 291
- US-A- 4 755 185

## Beschreibung

Zum Ersatz des Sprunggelenks ist eine Endoprothese bekannt, die aus einer mit dem Sprungbein zu verbindenden Komponente, einer mit dem Schienbein zu verbindenden Komponente und einem Zwischenteil besteht (DE-U-8812806; Prospekt "LINK S.T.A.R. Totale Sprunggelenkprothese [H. Kofoed] der Firma Waldemar Link (GmbH & Co.), Hamburg). Die Sprungbeinkomponente und der Zwischenteil wirken über zylindrische Gleitflächen zusammen, die Beugung und Streckung in der Sagittalebene ermöglichen. Die Schienbeinkomponente und der Zwischenteil bilden zusammenwirkende Gleitflächen, die eine Rotation um die Hochachse erlauben. Sie können eben ausgeführt sein, um Ausgleichsbewegungen in AP- und LM-Richtung zu ermöglichen. Die Stabilisierung geschieht durch den natürlichen Bandapparat.

Die ober- und unterseitigen Gleitflächen des Zwischenteils sind bei der bekannten Prothese in der Frontalebene parallel zueinander orientiert. Dies ist insofern konsequent, als die natürlichen Gleitflächen des Sprungbeins und des Schienbeins durch die prothetischen Gleitflächen ersetzt werden sollen, ohne daß damit eine Richtungsänderung verbunden ist. Jedoch hat sich gezeigt, daß die lateralen und medialen Bänder des Gelenks nach der Operation nicht selten eine unterschiedliche Spannung aufweisen, woraus Beschwerden resultieren können.

Die Erfindung ermöglicht es, diese Unausgewogenheit dadurch zu vermeiden, daß ein Zwischenteil zur Verfügung gestellt wird, der in der Frontalebene keilförmig ausgebildet ist. Die in der Frontalebene liegenden Richtungsachsen der Gleitflächen laufen nicht parallel zueinander, sondern in einem Winkel, der normalerweise zwischen 1° und 12° liegt. Je nach den vorgefundenen Verhältnissen kann der Arzt bestimmen, ob und welchen Winkel er zwischen den Gelenkachsen durch Auswahl des geeigneten Zwischenstücks vorsehen will. Dies kann gegebenenfalls auch intraoperativ geschehen.

Zwar ist eine Srunggelenkendoprothese bekannt (EP-A-1097680), bei welcher die zwischen der Sprungbeinkomponente und dem Zwischenteil befindlichen Gleitflächen Teile einer Kegelfläche bilden. Jedoch geht daraus nicht hervor, wie diese Gleitflächen in der Frontalebene gelegen sind.

Damit der Zwischenteil hinsichtlich seiner Keilform richtig orientiert bleibt, wird seine Ausrichtung zweckmäßigerweise zwangsweise vom Sprungbein oder vom Schienbein her festgelegt, indem die zusammenwirkenden Gleitflächenpaare entsprechend richtungsbestimmend ausgebildet sind. Dazu ist besonders das Gelenk zwischen dem Sprungbein und dem Zwischenteil geeignet.

Die Patentschrift FR-A-2676917 offenbart eine Sprunggelenkondoprothese mit verschiedenen Zwischenteilen.

Die Erfindung wird im folgenden näher unter Bezugnahme auf die Zeichnung erläutert, die ein vorteilhaftes Ausführungsbeispiel veranschaulicht. Es zeigen:
- Fig. 1: einen Sagittalschnitt durch das mit der Prothese versorgte Gelenk,
- Fig. 2: die Prothese perspektivisch aufgefächert,
- Fig. 3: einen Frontalschnitt durch den Zwischenteil und
- Fig. 4: einen perspektivischen Blick in die Anordnung der Knochen und zugehörigen Prothesenteile vor dem Einsetzen des Zwischenteils.

Zwischen dem Schienbein 1 und dem Sprungbein 2 ist die Prothese anzuordnen, die aus der Schienbeinkomponente 3, der Sprungbeinkomponente 4 und dem Zwischenteil 5 besteht. Die Schienbeinkomponente 3 weist einen plattenförmigen Teil 6 auf, dessen Unterseite 7 eine ebene Gleitfläche bildet. Vorsprünge 8 dienen zur Befestigung in entsprechenden Resektionsausnehmungen 9 des Schienbeins 1.

Die Sprungbeinkomponente 4 bildet eine konvex gewölbte Gleitfläche 10, die zylindrisch ausgebildet ist. Sie trägt eine Rippe 11, die in der Richtung der Relativbewegung des Zwischenteils bei der Beuge- und Streckbewegung liegt. Ferner weist die Sprungbeinkomponente seitliche Facetten 12 zum Zusammenwirken mit entsprechenden Gleitflächen des Schienbeins 1 und des Wadenbeins 13 auf.

Der Zwischenteil 5 besitzt eine ebene, zur Gleitfläche 7 passende Oberseite 15 und eine unterseitige Gleitfläche 16, die kongruent zur Gleitfläche 10 der Sprungbeinkomponente 4 ausgebildet ist. Sie enthält eine Nut 17 zur Aufnahme der Rippe 11. Dadurch wird der Zwischenteil 5 seitlich im Verhältnis zur Sprungbeinkomponente 4 geführt. Nur Beuge- und Streckbewegungen sind ihm erlaubt.

Die Komponenten 3 und 4 bestehen zweckmäßigerweise aus Metall und der Zwischenteil 5 aus gleitgünstigem Kunststoff wie Polyethylen. Jedoch sind auch andere Werkstoffe mit hinreichender Festigkeit und Gleitfähigkeit verwendbar, beispielsweise Keramik.

In Folge der kongruenten Form der Gleitflächen 10 und 16 sowie durch die Rippe 11 im Zusammenwirken mit der Nut 17 ist der Zwischenteil 5 bezüglich der Hochachse gegenüber der Sprunggelenkkomponente 4 nicht drehbar. Seine Ausrichtung ist also durch diejenige der Sprungbeinkomponente festgelegt. Während die gezeigte Ausführungsform derartige Rotationsbewegungen vollständig ausschließt, sind auch Ausführungen denkbar, in denen sie durch die Ausbildung der Gleitflächen lediglich gehemmt ist, indem diese beispielsweise elipsoidisch ausgebildet sind.

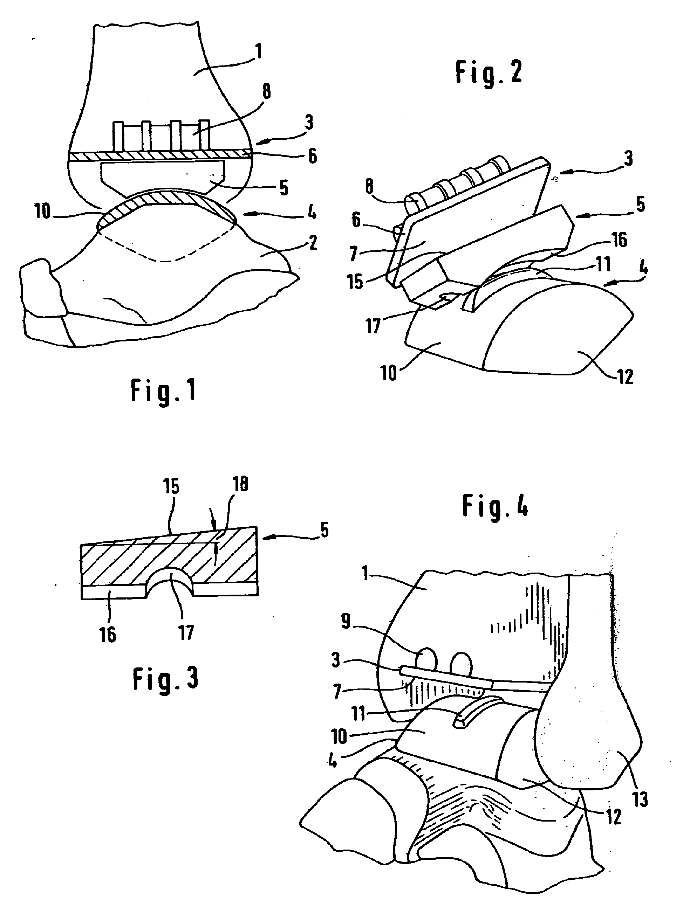

Im Frontalschnitt gemäß Fig. 3 ist der Zwischenteil keilförmig ausgebildet. Seine obere Gleitfläche 15 schließt mit der Richtung der unteren Gleitfläche 16 einen Keilwinkel 18 ein, der vorzugsweise zwischen 1° und 12° liegt. In den meisten Fällen liegt er zwischen 3° und 8°.

Sobald der Operateur, wie es in Fig. 4 gezeigt ist, die Schienbeinkomponente 3 und die Sprungbeinkomponente 4 implantiert hat, kann er mittels geeigneter Instrumente feststellen, ob deren Gleitflächen 7 bzw. 10 in LM-Richtung bei gespannten Seitenbändern parallel oder winklig zueinander verlaufen. Dem entsprechend wählt er einen geeigneten Zwischenteil 5 aus.

## Patentansprüche

1. Endoprothese zum Ersatz des Sprunggelenks bestehend aus einer mit dem Sprungbein (2) zu verbindenden Komponente (4), die eine oberseitige, zylindrische Gleitfläche (10) bildet, einer mit dem Schienbein (1) zu verbindenden Komponente (3), die eine unterseitige Gleitfläche (7) bildet, und einem Zwischenteil (5), der zwei Gleitflächen (15, 16) bildet, die mit den Gleitflächen (7, 10) der vorgenannten Komponenten derart zusammenwirken, daß seine Frontalrichtung festgelegt ist, **dadurch gekennzeichnet, daß** der Zwischenteil (5) in der Frontalebene keilförmig ist.

2. Endoprothese zum Ersatz des Sprunggelenks nach Anspruch 1, **dadurch gekennzeichnet, daß** die an der Sprungbeinkomponente (4) und dem Zwischenteil (5) zusammenwirkenden Gleitflächen (10, 16) im wesentlichen unrotierbar bezüglich der Hochachse zusammenwirken.

3. Endoprothese nach Anspruch 1, **dadurch gekennzeichnet, daß** die an der Schienbeinkomponente (4) und dem Zwischenteil (5) zusammenwirkenden Gleitflächen (7, 15) bezüglich der Hochachse rotierbar zusammenwirken.

4. Endoprothese nach einem der Ansprüche 1 bis 3, **dadurch gekennzeichnet, daß** der Keilwinkel (18) zwischen 1° und 12° liegt.

5. System von Endoprothesen zum Ersatz des Sprunggelenks, die aus einer mit dem Sprungbein (2) zu verbindenden Komponente (4), die eine oberseitige Gleitfläche (10) bildet, einer mit dem Schienbein (1) zu verbindenden Komponente (3), die eine unterseitige Gleitfläche (7) bildet, und einem Zwischenteil (5) bestehen, der zwei Gleitflächen (15, 16) bildet, die mit den Gleitflächen der vorgenannten Komponenten derart zusammenwirken, daß seine Frontalrichtung festgelegt ist, **dadurch gekennzeichnet, daß** unterschiedliche Zwischenteile zur Verfügung stehen, die im Frontalschnitt unterschiedliche Keilwinkel zwischen der oberen Gleitfläche (15) und der unteren Gleitfläche (16) aufweisen.

## Claims

1. An endoprosthesis for replacement of the ankle joint, consisting of a component (4) which is to be connected to the anklebone (2) and which forms an upper, cylindrical slide surface (10), of a component (3) which is to be connected to the tibia (1) and which forms a lower slide surface (7), and of a middle part (5) which forms two slide surfaces (15, 16) which interact with the slide surfaces (7, 10) of the aforementioned components in such a way that its frontal direction is fixed, wherein the middle part (5) is wedge-shaped in the frontal plane.

2. The endoprosthesis for replacement of the ankle joint as claimed in claim 1, wherein the interacting slide surfaces (10, 16) on the anklebone component (4) and the middle part (5) interact substantially nonrotatably with respect to the vertical axis.

3. The endorposthesis as claimed in claim 1, wherein the interacting slide surfaces (7, 15) on the tibial component (4) and the middle part (5) interact rotatably with respect to the vertical axis.

4. The endoprosthesis as claimed in one of claims 1 through 3, wherein the wedge angle (18) is between 1° and 12°.

5. A system of endoprostheses for replacement of the ankle joint, said endoprostheses consisting of a component (4) which is to be connected to the anklebone (2) and which forms an upper slide surface (10), of a component (3) which is to be connected to the tibia (1) and which forms a lower slide surface (7), and of a middle part (5) which forms two slide surfaces (15, 16) which interact with the slide surfaces of the aforementioned components in such a way that its frontal direction is fixed, wherein different middle parts are provided which, in frontal section, have different wedge angles between the upper slide surface (15) and the lower slide surface (16).

## Revendications

1. Endoprothèse pour remplacer l'articulation tibio-tarsienne, constituée d'un composant (4) à relier avec l'os de la cheville (2) formant une surface de glissement supérieure cylindrique (10), d'un composant (3) à relier avec le tibia (1), formant une surface de glissement inférieure (7) et d'un élément intermédiaire (5), formant deux surfaces de glissement (15, 16) qui coopèrent avec les surfaces de glissement (7, 10) des composants précédemment cités de façon à fixer sa direction frontale, **caractérisée en ce que** l'élément intermédiaire (5) est cunéiforme dans le plan frontal.

2. Endoprothèse pour remplacer l'articulation tibio-tarsienne selon la revendication 2, **caractérisée en ce que** les surfaces de glissement (10, 16) coopérant sur le composant pour l'os de la cheville (4) et l'élément intermédiaire (5) coopèrent de façon sensiblement non rotative par rapport à l'axe normal.

3. Endoprothèse selon la revendication 1, **caractérisée en ce que** les surfaces de glissement (7, 15) coopérant sur le composant pour le tibia (4) et sur l'élément intermédiaire (5) coopèrent de façon rotative par rapport à l'axe normal.

4. Endoprothèse selon l'une quelconque des revendications 1 à 3, **caractérisée en ce que** l'angle de bord d'attaque (18) est compris entre 1° et 12°.

5. Système d'endoprothèses pour remplacer l'articulation tibio-tarsienne, constituée d'un composant (4) à relier avec l'os de la cheville (2) formant une surface de glissement supérieure cylindrique (10), d'un composant (3) à relier avec le tibia (1), formant une surface de glissement inférieure (7) et d'un élément intermédiaire (5), formant deux surfaces de glissement (15, 16) qui coopèrent avec les surfaces de glissement des composants précédemment cités de façon à fixer sa direction frontale, **caractérisé en ce que** différents éléments intermédiaires sont disponibles, qui en coupe frontale présentent différents angles de bord d'attaque entre la surface de glissement supérieure (15) et la surface de glissement inférieure (16).
